# EUROPEAN PATENT APPLICATION

(11) **EP 4 763 971 A1**
(43) Date of publication of application: **24.06.2026**
(21) Application number: 24854430.6
(22) Date of filing: 12.08.2024
(51) Int. Cl.: C12M 3/06, C12M 3/00, C12N 5/077, C12N 5/071, G01N 33/50

(54) **MULTI-ORGANS-ON-A-CHIP WITH INVERTED MICROWEIR STRUCTURE AS MULTI-CELL CULTURE PLATFORM AND USE THEREOF**

(30) Priority: 14.08.2023 KR 20230106433
(71) Applicant: Humanase, Pocheon-si Gyeonggi-do 11160 (KR)
(72) Inventor: BAEK, Sangwon, Seongnam-si, Gyeonggi-do 13372 (KR); PARK, Sungbin, Seongnam-si, Gyeonggi-do 13357 (KR); KIM, Hyeonji, Seongnam-si, Gyeonggi-do 13311 (KR)
(74) Representative: Kador & Partner Part mbB
(86) International application number: PCT/KR2024/011954
(87) International publication number: WO 2025/037879

(57) **Abstract**

The present invention relates to an organ-on-a-chip comprising an inverted micro-weir structure as a cell co-culture platform, and uses thereof. By using the multi-organ-on-a-chip provided by the present invention, more accurate efficacy and toxicity results on cells in response to drug treatment can be derived. In particular, when a drug is converted into a metabolite through hepatic metabolism in a liver-heart co-culture system, the resulting cardiotoxicity and drug responses at a multi-organ level can be predicted. Utilizing this as an alternative testing method to animal testing may significantly reduce the cost and time required for novel drug development and drug screening, as well as being usefully applied in studies on intracellular microenvironments and other organ-on-a-chips.

## Description

### [Technical Field]

The present invention relates to a multi-organ-on-a-chip comprising an inverted micro-weir structure as a multi-cell culture platform, and uses thereof.

### [Background Art]

Drug development is time-consuming, intensive, and costly, but only a few developed drugs are approved. The costs and time for achieving regulatory approval for a single drug are greater than $2.5 billion and between 10 and 15 years, respectively. To address these issues, numerous animal- or cell-based experiments have been typically conducted during the initial stages of drug development.

Animal-based experiments are expensive, require a long time, and are not free from ethical issues, and in many cases, animal experimental results cannot be applied to humans due to the limitations of inter-species differences, such as the existence of different ion channels from humans and pharmacokinetic differences. Moreover, even drugs that have passed clinical trials through animal experiments are often withdrawn due to side effects. In cell-based experiments, cells are usually cultured under two-dimensional conditions. While two-dimensional cell culture models are advantageous in terms of ease of handling and high throughput, they have limitations in drug efficacy and toxicity evaluation since cells lose their inherent functionality during two-dimensional culture, resulting in incorrect evaluation results. In human organs, cells exist in a three-dimensional structure environment with extracellular matrix (ECM), and continuously receive nutrients and oxygen through diffusion from capillaries and diffuse and discharge the resultant wastes, metabolites, and carbon dioxide through blood vessels. Accordingly, there is a need for an efficient drug efficacy and toxicity evaluation platform that reflects the structural, physiological, and environmental characteristics of actual organs.

Organ-on-a-chips (OoCs) are systems that mimic the internal microenvironment structures of specific organs in the human body and allow cells constituting the corresponding organs to be cultured in micro-chambers, thereby implementing the characteristics thereof. Therefore, organ-on-a-chips are an effective alternative that can overcome the limitations of animal testing and are expected to serve as a pre-clinical new drug development platform by implementing physiological activity at the tissue or organ level.

Recently, research on multi-organ-on-a-chips has been attempted in line with the trend of focusing on the pharmacodynamic effects resulting from multi-organ interactions in drug efficacy and toxicity evaluation, but such research still remains in its early stages (Korean Patent No. 10-2281857; Korean Patent No. 10-2325876; Korean Patent Application Publication No. 10-2023-0110031; Korean Patent Application Publication No. 10-2023-0110153). Most of these studies simply co-culture component cells of multiple organs in two dimensions to determine the effectiveness of a circulatory system, without implementing the key microstructures of each organ. To overcome this, there is a need to develop a system capable of implementing the key microstructures of two or more organs and co-culturing the same. Accordingly, there is a need to develop an innovative multi-organ chip capable of mimicking the key microstructures of each organ and reflecting the pharmacokinetic effects of a drug resulting from interactions between organs.

The liver metabolizes drugs to remove them from the body, thereby causing changes in the function and bioavailability of the drug, which can generate drug metabolites that are more toxic or less toxic than the original drug. To this end, there is growing interest in *ex vivo* models that simultaneously test drug-related effects on multiple organs, including the liver.

### [Disclosure]

### [Technical Problem]

To accurately evaluate cardiotoxicity, which accounts for 40% of drug withdrawals, there is a growing need for the development of a low-cost, rapid, and accurate drug toxicity evaluation platform capable of replacing animal testing, in which the key microstructures of the liver and heart are implemented and interconnected, and hepatocytes and cardiomyocytes are organically co-cultured to allow drugs to be introduced to the heart through the liver.

### [Technical Solution]

A main object of the present invention is to provide a multi-organ-on-a-chip capable of culturing cells derived from different organs, comprising a medium supply unit for supplying medium to cells, a cell supply unit comprising two types of fixed cells, and an inverted micro-weir structure equipped therebetween.

Another object of the present invention is to provide a method for evaluating the toxicity of a drug on cells using the multi-organ-on-a-chip.

Still another object of the present invention is to provide a use of a multi-organ-on-a-chip for evaluating the toxicity of a drug on cells.

### [Advantageous Effects]

By using the multi-organ-on-a-chip provided by the present invention, more accurate efficacy and toxicity results on cells in response to drug treatment can be derived. In particular, when a drug is converted into a metabolite through hepatic metabolism in a liver-heart co-culture system, the resulting cardiotoxicity and drug responses at a multi-organ level can be predicted. Utilizing this as an alternative testing method to animal testing may significantly reduce the cost and time required for novel drug development and drug screening, as well as being usefully applied in studies on intracellular microenvironments and other organ-on-a-chips.

### [Brief Description of Drawings]

FIG. 1 is a schematic diagram including a three-dimensional view, a microstructure view, and chip constituents of a liver-heart-on-a-chip comprising hepatocytes and cardiomyocytes, which is an example of a multi-organ-on-a-chip.
FIG. 2 is a three-dimensional view of the liver-heart-on-a-chip.
FIG. 3 is a plan view of the liver-heart-on-a-chip.
FIG. 4 is a partially enlarged view of cell chambers and medium channels of the liver-heart-on-a-chip.
FIG. 5 is a partially enlarged view of inverted micro-weir structures and cell chambers of the liver-heart-on-a-chip.
FIG. 6 is an actual photograph of the liver-heart-on-a-chip, and enlarged photographs of a part showing food dyes injected into each chamber inside the chip, as well as of the cell culture results.
FIG. 7 is a schematic diagram illustrating the hepatic metabolism of cyclophosphamide.
FIG. 8 is a graph illustrating the comparative analysis of the beating rate of cardiomyocytes after treating a liver-heart-on-a-chip with cyclophosphamide (CPA), compared with the control group.

### [Detailed Description of Preferred Embodiments]

One aspect of the present invention to achieve the foregoing objects of the present invention provides a multi-organ-on-a-chip, comprising: (a) a medium supply unit comprising a medium supply chamber, a medium recovery chamber, and a medium channel communicating with each of the chambers; (b) a cell supply unit for co-culture comprising a cell inlet equipped at one end, a residue outlet equipped at the other end, and multiple cell culture sets composed of cell chambers adjacently located between the cell inlet and the residue outlet, wherein the multiple cell chambers extend in the same direction as the medium channel, wherein each of the multiple cell chambers is capable of fixing and culturing different cells, and wherein the multiple cell chambers are characterized by being adjacent to each other or to the medium channel, such that culture medium derived from the medium channel is capable of flowing into each of the cell chambers; and (c) an inverted micro-weir structure equipped in an area selected from the group consisting of an adjacent area between the medium channel and the cell chamber, an adjacent area between the cell chamber and the residue outlet, an adjacent area between the cell chambers, and a combination thereof.

A material for the general structure of the multi-organ-on-a-chip provided by the present invention is not particularly limited as long as it does not hinder or adversely affect the fixation and culture of cells. Examples of the material may be polycaprolactone (PCL), poly(dimethylsiloxane) (PDMS), polylactic acid (PLA), polyglycolic acid (PGA), polydioxanone (PDO), *etc.* Other examples of the material may be PDMS, *etc.,* which is optically transparent, highly durable, bio-friendly, and flexible.

The medium supply unit comprised in the multi-organ-on-a-chip provided by the present invention comprises a medium supply chamber, a medium recovery chamber, and a medium channel. Each of these constituents is described in detail as follows.

First, the medium supply chamber comprised in the medium supply unit serves to store a medium for cell culture, which is to be supplied to multiple cell chambers; and to supply the stored medium through a medium channel to each cell that is fixed and cultured in the multiple cell chambers.

Next, the medium recovery chamber comprised in the medium supply unit is a section where the medium supplied by the medium supply chamber is finally stored after passing through the medium channel. The medium recovery chamber serves to recover the medium contaminated after cell culture, and is used to prevent the contamination of surrounding environments due to the medium contaminated with various metabolites and wastes secreted from the cells after cell culture.

Finally, the medium channel comprised in the medium supply unit serves as a main flow path of the medium through which the medium circulates from the medium supply chamber to the medium recovery chamber. The medium channel serves to supply medium to each cell fixed and cultured in the multiple cell chambers, and to recover the medium used in cell culture. In the present invention, the medium channel may be interpreted as a constituent mimicking capillaries for supplying nutrients to cells that exist in a living body. Therefore, the width of the medium channel is not particularly limited as long as the medium channel can mimic the functions of capillaries. In one example, the medium channel may have a width of 20, 30, 40, 50, 60, 70, 80, 90, 100, 110, 120, 130, 140, 150, 160, 170, 180, 190, or 200 µm; in another example, the medium channel may have a width of 25, 30, 35, 40, 45, 50, 55, 60, 65, 70, 75, 80, 85, 90, 95, or 100 µm; and in still another example, the medium channel may have a width of 30 µm.

Moreover, vascular endothelial cells may be cultured inside the medium channel to prepare a multi-organ-on-a-chip in a form that is more similar to a living environment.

Each of the multiple cell culture sets comprised in the cell supply unit for co-culture comprises a cell inlet, a residue outlet, and cell chambers. Each of these constituents is described in detail as follows.

The cell supply unit for co-culture provided by the present invention comprises multiple cell culture sets, wherein each of the cell culture sets comprises an inlet, a residue outlet, and cell chambers. The cell chambers can be used to independently fix and culture cells, and therefore, the cell supply unit for co-culture can comprise different cells, each independently cultured by the multiple cell culture sets.

The cell inlet comprised in the cell culture sets serves to introduce a cell mixture, which is to be supplied to the cell chambers, and for example, may be configured such that the nozzle of a syringe may be connected to the cell inlet loaded with a cell mixture. The cell mixture may comprise cells and an auxiliary ingredient for assisting the flow and fixation of the cells. The cell chamber comprises cells that are arranged and exist in a fixed form therein, and an auxiliary ingredient is required for the injection of the cells to the cell chamber and the fixation of the injected cells inside the cell chamber. The auxiliary ingredient mimics the extracellular matrix (ECM) existing in the microenvironment around cells, is essential for three-dimensional cell culture, and serves to maintain the structures and functions of cultured cells to be similar to those *in vivo.* The auxiliary ingredient is not particularly limited as long as it can assist the introduction and fixation of cells. In one example, a hydrogel may be used, and in another example, collagen hydrogel, alginate hydrogel, GelMa hydrogel, *etc.* may be used. The hydrogel is mixed with cells to assist with three-dimensional culture of the cells, and additionally imparts fluidity to the cells to allow the cells to move from the cell inlet to the cell chamber. Furthermore, the hydrogel may be cured under various conditions. For example, collagen hydrogel may be cured by being left in an incubator for at least 1 hour to raise the temperature; alginate hydrogel may be cured by injecting a CaCl₂ solution into the medium channel in place of the medium; and GelMa hydrogel may be cured by ultraviolet ray irradiation. After the cell mixture moves to the cell chamber, the hydrogel contained therein is cured such that the cells comprised in the cured hydrogel can be naturally fixed. The inherent porous property of the hydrogel enables the delivery of external medium to the cells through the hydrogel, thereby assisting the culture of fixed cells.

As described above, the cell chambers comprised in the cell culture sets can store and fix the cell mixture supplied through the cell inlet, and serve as a culture container for culturing the fixed cells. An inverted micro-weir structure is equipped within a section of the cell chamber that communicates with the residue outlet, such that the cell mixture supplied through the cell inlet remains in the cell chamber without being discharged through the residue outlet.

In the present invention, the cell chamber may be interpreted as a constituent mimicking the microstructure of cells that exist in a living body. Therefore, the width of the cell chamber is not particularly limited as long as it can perform fixation and culture of cells required for co-culture. In one example, the width may be 100, 200, 300, 400, 500, 600, 700, 800, 900, 1000, 1100, 1200, 1300, 1400, 1500, 1600, 1700, 1800, 1900, or 2000 µm; in another example, the width of the cell chamber for culturing cardiomyocytes may be 150, 200, 250, 300, 350, 400, 450, 500, 550, 600, 650, 700, 750, 800, 850, 900, 950, or 1000 µm; and in still another example, the width of the cell chamber for culturing hepatocytes may be 100, 200, 300, 400, 500, 600, 700, 800, 900, 1000, 1100, 1200, 1300, 1400, 1500, 1600, 1700, 1800, 1900, or 2000 µm.

Furthermore, cell chambers comprised in one cell culture set are configured to be adjacent to cell chambers comprised in another cell culture set. An inverted micro-weir structure is equipped in the adjacent area between the cell chambers, thereby allowing the movement of medium between cell chambers, while preventing the movement of cells therebetween.

Meanwhile, the type of cells that can be injected into each of the cell chambers is not particularly limited. For example, the cells may be cardiomyocytes, cancer cells, smooth muscle cells, striated muscle cells, kidney cells, hepatocytes, epithelial cells, nerve cells, stem cells, vascular endothelial cells, scalp cells, splenocytes, pneumocytes, oral cells, skin cells, hair follicle cells, brain cells, spinal cord cells, *etc.* Different cells may be injected into each of the distinct cell chambers.

The residue outlet comprised in the cell culture set can serve to discharge contaminants, such as metabolites or wastes generated from the cells that are fixed and cultured in the cell chamber, to the outside. Such discharge of contaminants may be performed through the cell inlet as well as the residue outlet. That is, the cell inlet, which has finished its role of introducing cells into the cell chambers, may serve to discharge contaminants generated during the culture of the cells fixed in the cell chamber to the outside.

Like in the foregoing medium recovery chamber, the shape of the residue outlet is not particularly limited as long as it can serve to discharge contaminants to the outside, such as various metabolites and wastes secreted from cells comprised in the cell mixture. However, the residue outlet may be configured in a form to which a tube or a nozzle of a syringe can be attached, in order to prevent the contamination of the cell's surrounding environment by the contaminants.

As used herein, the term "inverted micro-weir structure" provided by the present invention refers to a partial blocking member equipped in an adjacent area between the medium channel and the cell chamber, an adjacent area between the cell chamber and the residue outlet, an adjacent area between the cell chambers, etc. The upper end of the inverted micro-weir structure is combined with the upper end of the adjacent area, and the lower end of the inverted micro-weir structure is spaced from the bottom of the adjacent area, thereby allowing the passage of a liquid component through the spaced site while preventing the passage of cells or cured cells therethrough (Korean Patent Application Publication No. 10-2023-0110031; Korean Patent Application Publication No. 10-2023-0110153).

The shape of the inverted micro-weir structure is not particularly limited as long as it exhibits the foregoing functionality, but may be a micro-barrier structure having a rectangular or arcuate cross-section, which prevents the cells from escaping the cell chambers while allowing only the passage of medium therethrough. Additionally, the gap of the spaced site equipped in the lower end is not particularly limited as long as it can prevent the passage of cells. In one example, the gap may be 5, 6, 7, 8, 9, 10, 11, 12, 13, 14, 15, 16, 17, 18, 19, or 20 µm; in another example, the gap may be 7, 8, 9, 10, 11, 12, 13, 14, or 15 µm; and in still another example, the gap may be 10 µm.

If the gap of the spaced site is less than 5 µm, a liquid component cannot pass between the lower end of the inverted micro-weir structure and the bottom of the adjacent area due to surface tension. If the gap of the spaced site is more than 20 µm, cells may move through the spaced site.

The multi-organ-on-a-chip provided by the present invention basically comprises at least one medium supply unit and at least one cell supply unit, but may comprise multiple medium supply units and cell supply units depending on the purpose of using the multi-organ-on-a-chip.

For example, the multi-organ-on-a-chip may comprise multiple medium supply units and one cell supply unit, one medium supply unit and multiple cell supply units, or multiple medium supply units and multiple cell supply units. In particular, if the multi-organ-on-a-chip comprises multiple medium supply units and one cell supply unit, each medium supply unit may be equipped at both sides of the cell supply unit. In such a case, the inverted micro-weir structure may be formed between multiple medium channels and one cell chamber, and in order to prevent the interference between each medium channel, each medium channel may be configured to avoid adjacency, or even if adjacent, to be completely isolated from each other.

According to one example of the present invention, the multi-organ-on-a-chip may be prepared in a form comprising (a) two medium supply units (medium supply chamber, medium channel, medium recovery chamber), and (b) a cell supply unit for co-culture composed of three cell culture sets (cell inlet, cell chambers, residue outlet).

In particular, it may be in a form in which, among the three cell chambers comprised in the three cell culture sets, hepatocytes are cultured in two of the cell chambers and cardiomyocytes are cultured in the remaining one cell chamber, and the two cell chambers culturing hepatocytes are arranged adjacent to each side of the one cell chamber culturing cardiomyocytes, in the longitudinal direction.

Furthermore, it may be in a form in which the two medium channels comprised in the two medium supply units are arranged adjacent to each of the outer sides of the two cell chambers culturing hepatocytes, in the longitudinal direction.

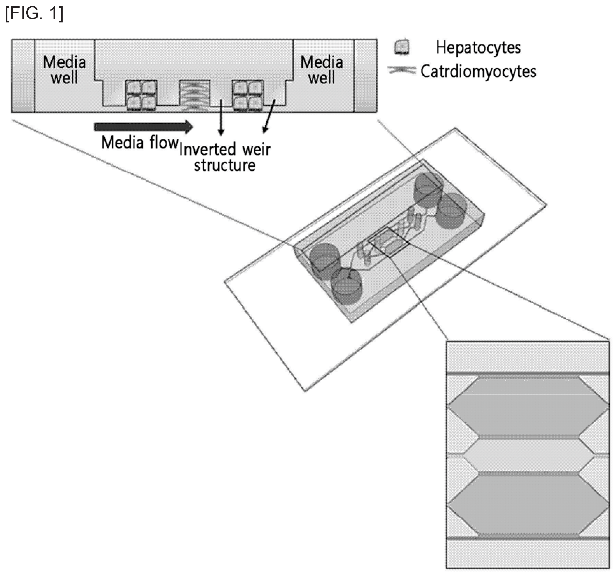

Hereinafter, the structure of the multi-organ-on-a-chip provided by the present invention will be described in more detail with reference to FIGs. 1 to 5 of the present invention.

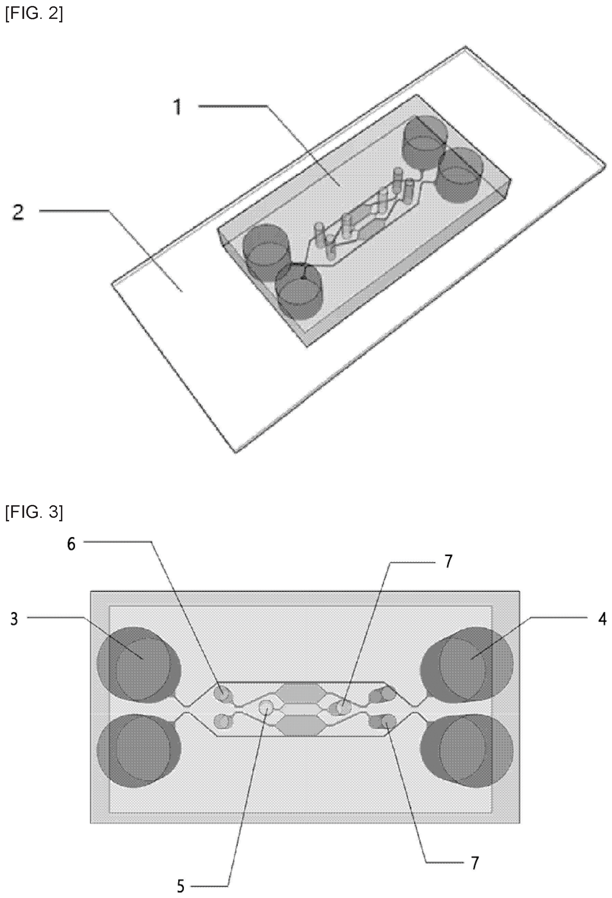

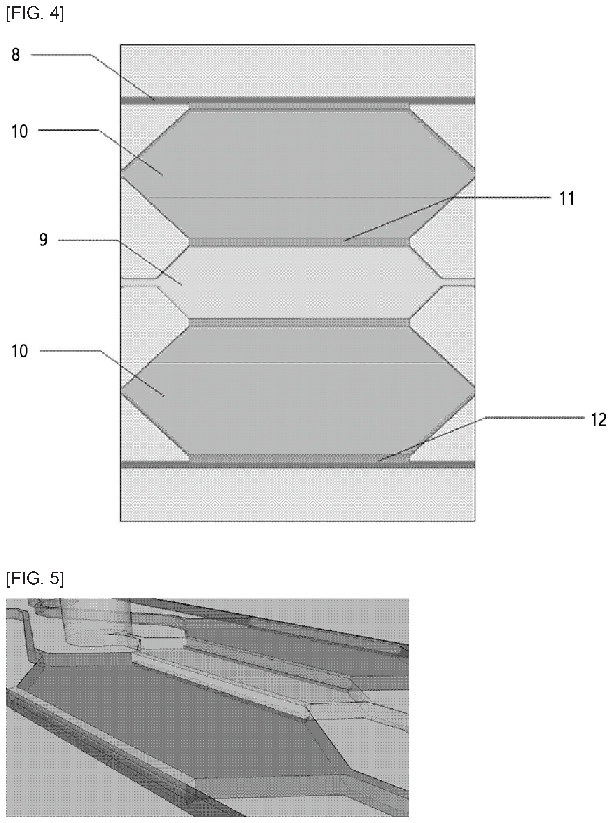

FIG. 1 is a schematic diagram including a three-dimensional view, a microstructure view, and chip constituents of a liver-heart-on-a-chip comprising hepatocytes and cardiomyocytes, which is an example of a multi-organ-on-a-chip; FIG. 2 is a three-dimensional view of the liver-heart-on-a-chip; FIG. 3 is a plan view of the liver-heart-on-a-chip; FIG. 4 is a partially enlarged view of cell chambers and medium channels of the liver-heart-on-a-chip; and FIG. 5 is a partially enlarged view of an inverted micro-weir structure and cell chambers of the liver-heart-on-a-chip.

The multi-organ-on-a-chip (1) provided by the present invention is in the form of a structure that is equipped on a slide glass (2), wherein two medium supply units each composed of a medium supply chamber (3), a medium recovery chamber (4), and a medium channel (8) are equipped on both sides of a cell supply unit comprising a cell inlet (5, 6), a residue outlet (7), and cell chambers (9, 10). The cell chamber (10) and the medium channel (8) are distinguished from each other by an inverted micro-weir structure (12) equipped in the adjacent area therebetween, and an inverted micro-weir structure (11) is also equipped in the adjacent area between the cell chamber (9) and another cell chamber (10).

Meanwhile, the multi-organ-on-a-chip can be used as a means to evaluate the efficacy of a drug.

Another aspect of the present invention provides a method for evaluating the efficacy or toxicity of a drug on cardiomyocytes using the multi-organ-on-a-chip.

As used herein, the term "drug efficacy or toxicity evaluation" refers to a series of actions to determine what positive effect (efficacy) or negative effect (toxicity) an externally administered drug has on cardiomyocytes after being processed through hepatocytes. The multi-organ-on-a-chip provided by the present invention may comprise, for example, two cell culture sets, and may be in the form of hepatocytes and cardiomyocytes cultured in each of the cell culture sets. In such form of a multi-organ-on-a-chip, when a drug is administered to the medium supply chamber, the administered drug is supplied to hepatocytes and metabolized therein, and may result in changes in the drug form. The drug thus changed may then be supplied to cardiomyocytes and induce a significant positive effect (efficacy) or negative effect (toxicity) therein. In particular, the drug administered to the medium supply chamber and the drug supplied to the cardiomyocytes may have the same form or different forms, and such form of a drug may be determined through hepatocyte metabolism. Accordingly, the multi-organ-on-a-chip provided by the present invention can be used for the purpose of determining how a desired drug is transformed through hepatocyte metabolism.

The method for evaluating the efficacy or toxicity of a drug on cardiomyocytes as provided by the present invention comprises: (a) culturing hepatocytes and cardiomyocytes in each of the multiple cell chambers comprised in the multi-organ-on-a-chip; (b) administering a desired drug to a medium supply chamber comprised in the multi-organ-on-a-chip; and (c) measuring changes in cardiomyocytes cultured in the cell chamber.

In particular, the change in the cardiomyocytes may be selected from but is not particularly limited to the group consisting of, for example, a change in the beating rate of the cultured cardiomyocytes, whether cardiomyocytes are damaged, whether cardiomyocytes grow, whether secreted components change, whether gene expression levels change, whether protein levels change, and a combination thereof. In another example, the change may be a change in the beating rate of the cultured cardiomyocytes.

According to one example of the present invention, hepatocytes (HepG2) and induced pluripotent stem cell-derived cardiomyocytes (Cardiosight-S) are injected and fixed into each of the two cell chambers comprised in the multi-organ-on-a-chip provided by the present invention, and cultured for 5 days to stabilize each cell. Then, when a drug whose toxicity changes through hepatic metabolism is administered to the medium supply chamber comprised in the multi-organ-on-a-chip, a change in the cardiomyocyte beating rate may occur due to the effect of the drug. Therefore, the effect of the drug on cardiomyocytes can be evaluated by examining whether there is a change in the beating rate of cardiomyocytes.

Still another aspect of the present invention provides a use of the multi-organ-on-a-chip for evaluating the efficacy or toxicity of a drug on cells.

### [Mode for Carrying Out the Invention]

Hereinafter, the present invention will be described in more detail by way of Examples. However, these Examples are given for illustrative purposes only, and the scope of the present invention is not intended to be limited by these Examples.

### Example 1: Preparation of multi-organ-on-a-chip

The multi-organ-on-a-chip of the present invention was prepared using a polydimethylsiloxane (PDMS) elastomer and a slide glass.

A mold that had completed PR patterning was prepared. In addition, a PDMS silicone base elastomer and a curing agent (SYLGARD 184 silicone elastomer kit, Dow Corning) were mixed at a ratio of 10:1, and then bubbles generated during the mixing were eliminated using a vacuum system of a plasma processing system (CUTE, FEMTO Science). Thereafter, the mixture was poured onto the SU-8 mold and solidified by heating on a hot plate at 80°C for 10 hours. The solidified product was separated from the mold, followed by cutting according to the appropriate size of a chip, and then punched out to form cell and hydrogel mixture inlet and outlet, and medium storage and inlet and outlet according to the size of each by using cylindrical biopsy punches with diameters of 1.2 mm and 8 mm, thereby preparing a microfluidic device.

The prepared PDMS-based microfluidic device was immersed in a sonicator containing isopropyl alcohol and washed by sonication, and then completely dried by a nitrogen air-gun.

The washed PDMS-based microfluidic device and a slide glass were dried in a dry oven at 80°C for 10 minutes and then sterilized by exposure to ultraviolet light for 30 minutes. Finally, the PDMS-based microfluidic device and the slide glass were placed in a plasma treatment system and were bound together by oxygen plasma treatment, thereby preparing an organ-on-a-chip. The prepared organ-on-a-chip was placed on a 35-mm petri dish, and was heated for 30 minutes on a hot plate heated to 80°C to enhance the adhesion from the plasma treatment.

The overall size of the multi-organ-on-a-chip prepared as described above was 3.7 cm in width and 1.9 cm in length, and the height of all the channel microstructures in the chip was 50 µm. The height excluding the inverted micro-weir structure was 5 µm. In addition, the width of each side cell chamber was 1700 µm, and the width of the middle cell chamber was 1000 µm. The width of medium channels was 30 µm, which is approximately three times the diameter of *in vivo* capillaries of about 10 µm.

In addition, the multi-organ-on-a-chip was configured to comprise three cell chambers, each of which was adjacent to each other and parallelly arranged in the longitudinal direction, such that one inner cell chamber is located between two outer cell chambers. Further, the medium channel was configured to be adjacent to the outside of the outer cell chamber.

An inverted micro-weir structure, positioned in the direction of the residue outlet of the cell chamber, was designed to have a thickness of 60 µm and be located in an arcuate shape in the direction of the outlet at a section immediately before the width of the cell chamber narrows, thereby inducing the injected cell-hydrogel mixture to accumulate without escaping through the residue outlet, consequently achieving smooth injection and enabling the release of only the pressure generated during the injection through the residue outlet. Additionally, inverted micro-weir structures equipped in adjacent areas between the cell chambers and the medium channels and in adjacent areas between the cell chambers were designed into a rectangular shape with a thickness of 100 µm, and similarly, the cell-hydrogel mixture was allowed to accumulate rather than escape the cell chamber. Each of the inlet and outlet connected to the cell chambers had a diameter of 1.5 mm, and the diameter of the medium channels connected to the medium supply chamber and medium recovery chamber was set to 6 mm.

### Example 2: Preparation of multi-organ-on-a-chip comprising hepatocytes and cardiomyocytes

First, 100 µl of medium was loaded into each of the medium supply chambers comprised in the multi-organ-on-a-chip prepared in Example 1. The flow of medium was connected between each chamber using a pipette, and then medium was filled inside the medium recovery chamber to ultimately form the medium flow. In particular, it was confirmed that the medium formed a boundary between the medium channel and the cell chamber due to surface tension, with the inverted micro-weir structure serving as a boundary.

Next, cardiomyocytes (Cardiosight-S) differentiated from human pluripotent stem cells (hPSCs) from NEXEL were mixed with a hydrogel such as collagen, and then injected using a pipette into the cell inlet connected to the inner cell chamber of the multi-organ-on-a-chip prepared in Example 1, thereby injecting cardiomyocytes into the inner cell chamber.

Next, 1 x 10⁵ cultured hepatocytes (HepG2) were injected using a pipette into each cell inlet connected to the two outer cell chambers of the multi-organ-on-a-chip prepared in Example 1, thereby injecting hepatocytes into the two outer cell chambers.

As a result, it was confirmed that hepatocytes and cardiomyocytes formed a boundary between cell chambers due to the inverted micro-weir structure, and that hepatocytes, cardiomyocytes, and culture medium were each successfully separately injected. It was confirmed that the time required for the injection process of a cardiomyocyte mixture using the organ-on-a-chip was less than 10 minutes per chip, and that these results were reproducible. This was confirmed to be a significantly reduced experimental time compared to the 3 hours per chip required for the injection process of a cardiomyocyte mixture using an existing perimysium-mimicking heart-on-a-chip.

Next, the hydrogel comprised in the injected cardiomyocyte mixture was cured to fix the cardiomyocytes within the cell chamber. Depending on the type of hydrogel, curing is performed using different curing methods. In the case of collagen, curing was performed via temperature change by placing in an incubator for at least 1 hour, and then cardiomyocytes were continuously cultured in the incubator.

Further, hepatocytes were fixed and cultured using the same method.

The culture was performed by supplying a culture medium comprising a mixture of Cardiosight-S Media supplemented with supplement (50X) and Minimum Essential Media Eagle (MEM, high-glucose, gibco) supplemented with 25 mM HEPES (4-(2-hydroxyethyl)-1-piperazineethanesulfonic acid, gibco), 10% (v/v) FBS, P/S (100 Uml-1 penicillin/100 Uml-1 streptomycin, Sigma-Aldrich Co., MO) at a certain ratio. The medium was replaced daily with fresh medium to provide continuous medium flow and fresh nutrients, and the existing media in medium supply chamber and recovery chamber were removed during medium replacement, thereby removing waste products while culturing.

Using the culture method, a multi-organ-on-a-chip comprising hepatocytes and cardiomyocytes (hereinafter referred to as "liver-heart multi-organ-on-a-chip") was prepared by culturing the cells in a humidified incubator at 37°C and 5% CO₂ for 5 days.

### Example 3: Evaluation of drug efficacy and toxicity using liver-heart multi-organ-on-a-chip

### Example 3-1 Evaluation of functionality of liver-heart multi-organ-on-a-chip

It was evaluated whether the liver-heart multi-organ-on-a-chip prepared in Example 2 functioned normally.

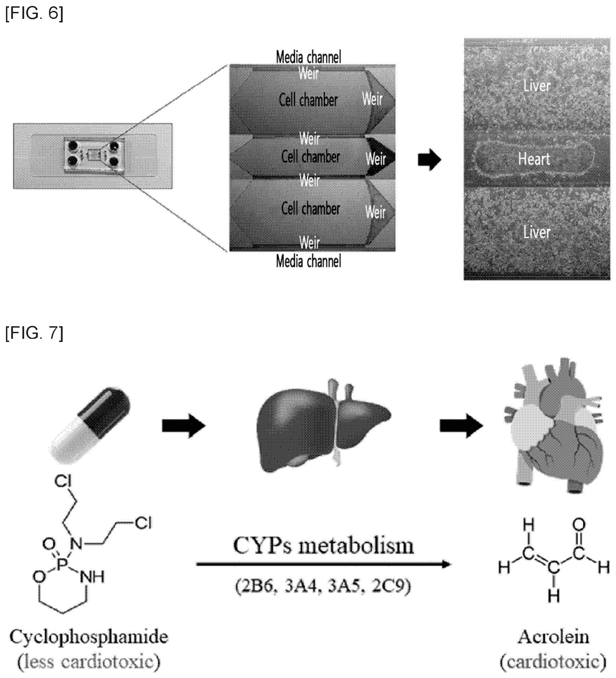

In brief, red, blue, and green food dyes were adjusted to appropriate concentrations and viscosities. A 100 µl of a red food dye solution was loaded into each of the medium supply chamber and recovery chamber comprised in the liver-heart multi-organ-on-a-chip, and then using a pipette, the food dye solution was filled into the inside of the medium channels located on both sides. Thereafter, 100 µL of dye solution was also loaded into the remaining set of medium supply chamber and recovery chamber, such that the flow of the red dye solution was connected between each chamber. Afterwards, a green food dye solution was injected into the cell chambers on both sides at a rate of 20 µl/min using a syringe pump (NE-1000, NEWERA), and likewise, a blue food dye solution was injected into the middle cell chamber at a rate of 20 µl/min using the same syringe pump (NE-1000, NEWERA) (FIG. 6).

FIG. 6 is an actual photograph of the liver-heart-on-a-chip, and enlarged photographs of a part showing food dyes injected into each chamber inside the chip, as well as of the cell culture results.

As shown in FIG. 6, each dye solution formed a boundary between the medium channel and the cell chamber due to surface tension with the inverted micro-weir structure serving as a boundary, thereby confirming that the selective liquid was successfully separately injected.

### Example 3-2 Evaluation of drug toxicity using liver-heart multi-organ-on-a-chip

The cardiomyocytes in the liver-heart multi-organ-on-a-chip prepared in Example 2 above begin to beat as early as 1 to 2 days of culture, and as the culture time passes, the beating rate increases. The culture period prior to drug efficacy and toxicity evaluation was set to 5 days based on the beats per minute (BPM), in consideration of achieving an optimal beating rate while maintaining optimal hepatocyte condition.

Meanwhile, cyclophosphamide (CPA) is known to be metabolized in the liver to produce acrolein, a cardiotoxic metabolite (FIG. 7).

FIG. 7 is a schematic diagram illustrating the hepatic metabolism of cyclophosphamide.

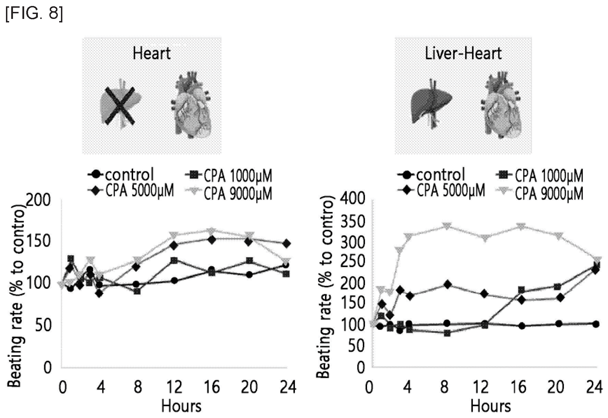

For drug efficacy evaluation of the liver-heart multi-organ-on-a-chip prepared in Example 2, cyclophosphamide was treated at various concentrations (0, 1,000, 5,000, or 9,000 µM), and the resulting changes in cardiomyocyte beating were observed using an optical microscope, and the cardiomyocyte beating rate was measured and analyzed (FIG. 8). In particular, as a control group, a heart-on-a-chip prepared by injecting only cardiomyocytes into the multi-organ-on-a-chip prepared in Example 1, without injecting hepatocytes, was used.

FIG. 8 is a graph illustrating the comparative analysis of the beating rate of cardiomyocytes after treating a liver-heart-on-a-chip with cyclophosphamide (CPA), compared with the control group.

As shown in FIG. 8, it was confirmed that cyclophosphamide had a more significant effect on the beating rate of cardiomyocytes in the liver-heart-on-a-chip, compared to the control heart-on-a-chip.

Specifically, when the control heart-on-a-chip was treated with 9,000 µM cyclophosphamide, the beating rate of the cardiomyocytes increased by 31.76 ± 0.59% compared to the beating rate of the cardiomyocytes not treated with cyclophosphamide. However, when the liver-heart-on-a-chip was treated with 9,000 µM cyclophosphamide, the beating rate of the cardiomyocytes increased by 177.9 ± 2.56% compared to the beating rate of the cardiomyocytes not treated with cyclophosphamide.

From these experimental results, it was confirmed that when hepatocytes and cardiomyocytes were co-cultured, cyclophosphamide, with weak cardiotoxicity, was transformed through liver metabolism into acrolein, a cardiotoxic substance, and the beating rate increased from abnormal cell function due to toxicity. These results were similar to those seen in existing clinical trials, and were determined to indicate the usefulness of the organ-on-a-chip for evaluating drug efficacy and toxicity.

From the above description, those skilled in the art to which the present invention pertains would understand that the present invention can be implemented in other specific forms without changing its technical concept or essential features. In this regard, the above-described examples should be understood as illustrative and not limited in any way. The scope of the present invention should be interpreted to include all modifications or modified forms derived from the meaning and scope of the claims as set forth below, as well as equivalent concepts, rather than from the detailed description above.

### Explanation of reference numerals

1 : PDMS-based microfluidic device
2 : slide glass
3 : medium supply chamber
4 : medium recovery chamber
5 : cardiomyocyte inlet
6 : hepatocyte inlet
7 : residue outlet
8 : medium channel
9 : cardiomyocyte chamber
10 : hepatocyte chamber
11 : inverted micro-weir structure located in adjacent area between cardiomyocyte chamber and hepatocyte chamber
12 : inverted micro-weir structure located in adjacent area between hepatocyte chamber and medium channel

## Claims

1. A multi-organ-on-a-chip, comprising: (a) a medium supply unit comprising a medium supply chamber, a medium recovery chamber, and a medium channel communicating with each of the chambers;
(b) a cell supply unit for co-culture comprising a cell inlet equipped at one end, a residue outlet equipped at the other end, and multiple cell culture sets composed of cell chambers adjacently located between the cell inlet and the residue outlet, wherein the multiple cell chambers extend in the same direction as the medium channel, wherein each of the multiple cell chambers is capable of fixing and culturing different cells, and wherein the multiple cell chambers are **characterized by** being adjacent to each other or to the medium channel, such that culture medium derived from the medium channel is capable of flowing into each of the cell chambers; and
(c) an inverted micro-weir structure equipped in an area selected from the group consisting of an adjacent area between the medium channel and the cell chamber, an adjacent area between the cell chamber and the residue outlet, an adjacent area between the cell chambers, and a combination thereof.

2. The multi-organ-on-a-chip according to claim 1, wherein the medium supply chamber stores a medium for cell culture, which is to be supplied to the cell chambers through the medium channel and the inverted micro-weir structure.

3. The multi-organ-on-a-chip according to claim 1, wherein the medium channel is a medium flow path from the medium supply chamber to the medium recovery chamber.

4. The multi-organ-on-a-chip according to claim 1, wherein the medium channel has a width of 20 to 200 µm.

5. The multi-organ-on-a-chip according to claim 1, wherein the medium channel has a form in which vascular endothelial cells are cultured therein.

6. The multi-organ-on-a-chip according to claim 1, wherein the cell inlet serves to introduce a cell mixture to be supplied to the cell chambers.

7. The multi-organ-on-a-chip according to claim 6, wherein the cell mixture comprises cells and a hydrogel for assisting the flow and fixation of the cells.

8. The multi-organ-on-a-chip according to claim 1, wherein the cell chamber serves as a culture container in which cells comprised in a cell mixture supplied through the cell inlet are arranged and fixed, and the fixed cells are cultured.

9. The multi-organ-on-a-chip according to claim 8, wherein the fixation of the cells is performed by curing the hydrogel comprised in the cell mixture.

10. The multi-organ-on-a-chip according to claim 8, wherein the culturing is performed such that the medium supplied from the medium channel through the inverted micro-weir structure is supplied to the cells fixed in the cell chamber.

11. The multi-organ-on-a-chip according to claim 8, wherein the cells to be cultured are selected from the group consisting of cardiomyocytes, smooth muscle cells, striated muscle cells, kidney cells, hepatocytes, epithelial cells, nerve cells, stem cells, organoids, pancreatic cancer cells, fibroblasts, vascular endothelial cells, scalp cells, splenocytes, pneumocytes, oral cells, skin cells, hair follicle cells, brain cells, spinal cord cells, and combinations thereof.

12. The multi-organ-on-a-chip according to claim 8, wherein the cells to be cultured are hepatocytes and cardiomyocytes.

13. The multi-organ-on-a-chip according to claim 1, wherein the cell chamber has a width of 100 to 2000 µm.

14. The multi-organ-on-a-chip according to claim 1, wherein the residue outlet serves to discharge contaminants, generated from the cells that are fixed and cultured in the cell chamber, to the outside.

15. The multi-organ-on-a-chip according to claim 1, wherein an upper end of the inverted micro-weir structure is combined with an upper end of the adjacent area, and a lower end of the inverted micro-weir structure is spaced from the bottom of the adjacent area, thereby allowing the passage of a liquid component through the spaced site while preventing the passage of cells therethrough.

16. The multi-organ-on-a-chip according to claim 15, wherein the spaced site has a gap of 5 µm to 20 µm.

17. The multi-organ-on-a-chip according to claim 1, wherein the cross-section of the inverted micro-weir structure is rectangular or arc-shaped.

18. The multi-organ-on-a-chip according to claim 1, wherein the inverted micro-weir structure equipped in the adjacent area between the medium channel and the cell chamber extends along the adjacent area in the same direction, and allows the interflow of culture medium between the medium channel and the cell chamber through the spaced site at the lower end.

19. The multi-organ-on-a-chip according to claim 1, wherein the inverted micro-weir structure equipped in the adjacent area between the cell chamber and the residue outlet is in the form of an arc curved toward the residue outlet, and allows the discharge of residue from the cell chamber to the residue outlet through the spaced site at the lower end.

20. The multi-organ-on-a-chip according to claim 1, wherein the inverted micro-weir structure equipped in the adjacent area between the cell chambers allows the interflow of culture medium between the cell chambers through the spaced site at the lower end.

21. The multi-organ-on-a-chip according to claim 1, wherein the multi-organ-on-a-chip comprises multiple medium supply units and one cell supply unit.

22. The multi-organ-on-a-chip according to claim 21, wherein the medium supply units are arranged on each side of the cell supply unit.

23. The multi-organ-on-a-chip according to claim 1, wherein the multi-organ-on-a-chip comprises one medium supply unit and multiple cell supply units.

24. The multi-organ-on-a-chip according to claim 1, wherein the multi-organ-on-a-chip comprises multiple medium supply units and multiple cell supply units.

25. The multi-organ-on-a-chip according to claim 1, wherein the multi-organ-on-a-chip comprises (a) two medium supply units and (b) a cell supply unit for co-culture composed of three cell culture sets.

26. The multi-organ-on-a-chip according to claim 24, wherein, among the three cell chambers comprised in the three cell culture sets, hepatocytes are cultured in two of the cell chambers and cardiomyocytes are cultured in the remaining one cell chamber, and the two cell chambers culturing hepatocytes are arranged adjacent to each side of the one cell chamber culturing cardiomyocytes, in the longitudinal direction; and the two medium channels comprised in the two medium supply units are arranged adjacent to each of the outer sides of the two cell chambers culturing hepatocytes, in the longitudinal direction.

27. Use of the multi-organ-on-a-chip according to any one of claims 1 to 26 for evaluating the efficacy or toxicity of a drug on cells.

28. A method for evaluating the efficacy or toxicity of a drug on cardiomyocytes, the method comprising: (a) culturing hepatocytes and cardiomyocytes in each of the multiple cell chambers comprised in the multi-organ-on-a-chip according to any one of claims 1 to 26;
(b) administering a desired drug to a medium supply chamber comprised in the multi-organ-on-a-chip; and
(c) measuring changes in cardiomyocytes cultured in the cell chamber.

29. The method according to claim 28, wherein (a) is performed using a multi-organ-on-a-chip comprising three cell culture sets, and performed such that among the three cell chambers comprised in the three cell culture sets, cardiomyocytes are cultured in the middle cell chamber, and hepatocytes are cultured in the two cell chambers adjacent to each side of the cell chamber culturing cardiomyocytes.

30. The method according to claim 28, wherein the change in the cardiomyocytes is selected from the group consisting of a change in the beating rate of the cultured cardiomyocytes, whether cardiomyocytes are damaged, whether cardiomyocytes grow, whether secreted components change, whether gene expression levels change, whether protein levels change, and a combination thereof.

31. The method according to claim 28, wherein the change in the cardiomyocytes is a change in the beating rate of the cultured cardiomyocytes.
